# EUROPEAN PATENT APPLICATION

(11) **EP 3 081 230 A1**
(43) Date of publication of application: **19.10.2016**
(21) Application number: 15163739.4
(22) Date of filing: 15.04.2015
(51) Int. Cl.: A61K 39/00, C12N 15/86

(54) **PHLEBOVIRUS-BASED VECTOR VACCINES FOR CANCER IMMUNOTHERAPY**

(71) Applicant: Stichting Dienst Landbouwkundig Onderzoek, 6708 PB Wageningen (NL)
(72) Inventor: Kortekaas, Jeroen Alexander, 8016 DZ Zwolle (NL); Oreshkova, Nadia Dimitrova, 8255 GJ Swifterbant (NL); Wichgers Schreur, Paulus-Jozef, 8096 MK Oldebroek (NL); Moormann, Robertus Jacobus Maria, 8252 EH Dronten (NL)
(74) Representative: V.O.

(57) **Abstract**

The invention relates to a Phlebovirus-based vector, whereby a gene encoding a tumor-associated antigen is functionally inserted into the genome of said Phlebovirus, and to methods of ameliorating and/or treating an individual suffering from a tumor, comprising administering said Phlebovirus-based vector to said individual.

## Description

The invention relates to the field of viruses, more specifically, to the field of molecular virology of Phleboviruses. The invention relates to a recombinant, avirulent Phlebovirus, especially for use in dendritic cell (DC)-mediated cancer immunotherapy.

Dendritic cells (DCs) are key players in the initiation and regulation of immune responses. Immature DCs are equipped with a broad range of pattern recognition receptors and are very effective in recognizing pathogen-associated molecular patterns (PAMPs). When contact with a PAMP occurs, DCs start to mature. During this process, the cells undergo changes in their morphology, migratory capability, expression of surface molecules and function (Prechtel and Steinkasserer, 2007. Arch Dermat Res 299: 59-69). The cells migrate from areas of antigen uptake to T-cell areas of secondary lymphoid organs, where they present antigen-derived peptides and instruct epitope-specific naive T-cells to develop their effector function (Reis and Sousa, 2006. Nature Rev Immunol 6: 476-483).

Vaccination strategies involving DCs have been developed to initiate and regulate efficient immune responses against tumor cells. To this end, DCs are provided with one or more tumor-specific antigens or immunogenic peptide(s) thereof. Considerable effort has been made to develop strategies to introduce tumor-specific antigens into DCs to initiate a specific immune response against the tumor-specific antigen. Peptide- or protein-loading of DCs is presently the predominant strategy to introduce tumor-specific antigens into DCs. The most important disadvantages of these approaches include poor efficiency of antigen presentation and the uncertainty regarding its quality and duration. By making use of viral vectors, antigen presentation is generally much more efficient and the abovementioned variables can be predicted with greater accuracy. This so-called viral transduction is therefore considered an attractive approach for engineering DCs to present tumor antigen-derived peptides (Kirk and Mule, 2000. Hum Gene Ther 11: 797-806). DCs can be transduced *in vitro* and be returned to the patient to allow the cells to perform their effector function. Several studies have, however, demonstrated that only a subset of *in vitro-*loaded DCs reach the lymph node to prime and activate naive T cells (Morse et al., 1999. Cancer Res 59: 56-58; De Vries, 2003. Cancer Res 63: 12-17), suggesting that this therapeutic approach requires optimization (Lesterhuis et al., 2008. Crit Rev Oncol Hematol 66: 118-134). Alternatively, virus vectors can be used for *in vivo* targeting of DCs.

Although numerous viruses have been used to deliver tumor antigens to DCs, the efficacy of T cell priming largely depends on how infected DCs as well as bystander DCs respond to the infection. Preferably, infected DCs produce a tumor-associated antigen or peptide(s) derived thereof, resulting in antigen-derived peptide presentation via MHC-I. Infected DCs are activated by the infection, resulting in their migration to T-cell areas of secondary lymphoid organs, where they present antigen-derived peptides via MHC-I to T-cells. Viruses that were previously evaluated for DC-mediated immunotherapy include members of the DNA viruses *Poxviridae, Herpesviridae, Adenoviridae, Parvoviridae* and the *Retroviridae.* Although several of these viruses have shown promise for cancer immunotherapy, RNA viruses offer several advantages over the use of DNA- or retroviruses. Considering that RNA viruses lack a DNA phase, there are no concerns about integration of viral genetic material into chromosomal DNA of the patient. Related to efficacy, DNA viruses, such as the poxviruses, encode hundreds of proteins. Immune responses against these proteins could negatively influence immune responses against vectored antigens and can even lead to a reduction in the overall memory T cell pool (Liu et al., 2003. J Virol 77: 7756-63). In addition, several poxvirus proteins were shown to modulate or antagonize host innate immune responses, which could further reduce vaccine effectiveness. Although RNA viruses also encode immune-modulating proteins, these genes can generally be easily mutated or deleted from the genome.

Among RNA viruses, those that naturally target DCs, such as the arboviruses, are particularly interesting candidate vectors for antigen delivery. Members of the positive-strand RNA virus families *Togaviridae* and *Flaviviridae* have been extensively evaluated for cancer immunotherapy (Anraku et al., 2002. J Virol 76: 3791-3799; Herd et al., 2004. Virology 319: 237-248; Moran et al., 2005. J Immunol 175: 3431-3438; Morse et al., 2010. J Clin Invest 120: 3234-3241), some of which have already entered clinical trials (Morse et al., 2010. J Clin Invest 120: 3234-3241). However, positive-strand RNA viruses are sensitive to mutation and recombination, which are disadvantages when using these viruses as vaccine vectors (Bukreyev et al., 2006 . J Virol 80: 10293-10306).

Therefore, the invention provides an avirulent Phlebovirus-based vector, whereby a gene encoding a tumor-associated antigen is functionally inserted into the genome of said Phlebovirus-based vector.

Negative-strand RNA viruses, such as Phleboviruses, are much less prone to both mutation and recombination, when compared to positive-strand RNA viruses such as Togaviridae and Flaviviridae, rendering these viruses more suitable for this approach.

Phleboviruses belong to the *Bunyaviridae* family, which have thus far not been used for introduction of tumor-specific antigens into DCs. The *Bunyaviridae* is a large family of viruses that are important pathogens of humans and livestock.

Bunyaviruses are negative-strand RNA viruses with three-segmented genomes (Elliott, R. M. 1996. The Bunyaviridae. Plenum Press, New York, NY). The *Bunyaviridae* family comprises five genera: Phlebovirus, Orthobunyavirus, Nairovirus, Hantavirus and Tospovirus. With the exception of the plant-infecting Tospovirus genus, bunyaviruses are pathogens of animals and humans. The small (S) Phlebovirus genome segment encodes a nucleocapsid (N) protein and, with the possible exception of the Nairovirus genus, a nonstructural protein named NSs. The NSs protein of animal-infecting bunyaviruses functions as an antagonist of host innate immune responses and is considered the major virulence factor. The medium-size (M) genome segment encodes the viral structural glycoproteins Gn and Gc and, depending on the genus, one or more nonstructural proteins referred to as NSm. The M segment of Rift Valley fever virus (RVFV, genus Phlebovirus) additionally encodes a 78-kDa protein believed to be a minor structural protein. The large (L) genome segment encodes the viral RNA-dependent RNA polymerase (RdRp). Bunyaviruses that cause serious disease in animals and/or humans are classified as biosafety level 3 (BSL-3) or 4 pathogens, complicating their use as vectors in cancer immunotherapy.

Among the negative-strand RNA viruses, those that contain segmented genomes are of particular interest. Genome segments of segmented negative-strand RNA viruses, including phleboviruses such as RVFV, form blunt-ended dsRNA panhandles which contain 5' triphosphates. This structure is an optimal ligand for the cytoplasmic pattern recognition receptor RIG-I, and is thereby an excellent inducer of adjuvanting innate immune responses (Schlee et al., 2009. Immunity 31: 25-34). Whereas the NSs protein of wildtype RVFV effectively counteracts these immune responses (Le May et al., 2008. PLoS Pathog 4: e13), infection of DCs with variants lacking the NSs protein results in strong interferon responses triggered by RIG-I signaling (Ermler et al., 2013. J Virol 87: 4846-4860).

The term "functionally inserted", as is used herein, indicates that a gene encoding a tumor-associated antigen is inserted into the genome of an avirulent Phlebovirus and that the gene is expressed in cells that are infected by said avirulent Phlebovirus.

In a preferred Phlebovirus-based vector of the invention, the gene encoding said tumor-associated antigen is expressed from the NSs locus, thereby preventing expression of wild type, full length NSs in infected cells. It is further preferred that the gene encoding said tumor antigen replaces a NSs encoding region, preferably the complete NSs encoding region, on the S-genome segment, thereby preventing expression of NSs in cells that are infected with a Phlebovirus-based vector of the invention. Elements for S-genome replication and expression of tumor antigen in infected cells are preferably provided by the UTRs at the 3' and the 5' end of the S genome segment.

The Phlebovirus genus comprises over 70 distinct serotypes, of which the majority is divided over two groups: the Phlebotomus fever viruses (the sandfly group, transmitted by *Phlebotominae* sandflies), comprising 55 members; and the Uukuniemi group (transmitted by ticks), comprising 13 members. Preferred viruses include Rift Valley fever virus (RVFV) and further members of the Phlebovirus genus: Toscana virus, Sandfly fever Naples virus, Sandfly fever Sicilian virus, Punta Toro virus, Uukuniemi virus, Massilia virus, severe fever with thrombocytopenia syndrome (SFTS) virus and Heartland virus.

The Phleboviruses Uukuniemi virus (UUKV) and Rift Valley fever virus (RVFV) were recently shown to infect immature DCs with high efficiency, using dendritic cell-specific intercellular adhesion molecule-3-grabbing non-integrin (DC-SIGN) as a receptor (Lozach et al., 2011. Cell Host Microbe 10: 75-88). In another study, RVFV was shown to specifically target cells of the monocyte/macrophage/dendritic cell lineages (Gommet et al., 2011. PLoS Negl Trop Dis 5: e1421). Hence, a further advantage of a Phlebovirus-based vector according to the invention is that it may infect professional antigen-presenting cells with high efficiency.

RVFV is an example of a Phlebovirus that is classified as a BSL-3 pathogen. RVFV is endemic to the African continent, Madagascar, the Comoros Islands, Mayotte and the Arabian Peninsula, where it is transmitted among livestock by mosquito vectors. RVFV epizootics are characterized by near simultaneous abortions, particularly among sheep, and high mortality among young animals below the age of two weeks. Humans can be infected via mosquito bite, but more commonly by inhalation of aerosols released during slaughtering of viremic animals. Infection of humans generally results in transient febrile illness. However, a small percentage of individuals develop complications such as retinal lesions, hepatic disease with hemorrhagic fever or delayed-onset encephalitis.

RVF vaccines of optimal efficacy and safety were recently described (Kortekaas et al., 2011. J Virol 85: 12622-12630; Wichers Schreur et al., 2015. Vaccine 33: 1459-1464).

A first vaccine is based on a nonspreading, genome replication-competent RVFV particle. These RVFV replicon particles (RRP), also known as nonspreading RVFV (NSR), lack a functional glycoprotein GnGc-encoding M segment. NSR particles are infectious for both mammalian and insect cells but are incapable of autonomous spreading (Kortekaas et al., 2011. J Virol 85: 12622-12630). A single intramuscular vaccination with NSR particles protected mice and lambs from a lethal dose of RVFV (Kortekaas et al., 2011. J Virol 85: 12622-12630; Kortekaas et al., 2012. Vaccine 30: 3423-3429; Oreshkova et al., 2013. PLoS One 8: e77461; Kortekaas et al., 2014. Vaccine 32: 4901-4908).

A second RVF vaccine is based on recently developed four-segmented RVF viruses (Wichers Schreur et al., 2015. Vaccine 33: 1459-1464). In these viruses, the Gn and Gc coding regions are functionally present on two separate genome segments. RVFV-4s has reduced replication capabilities in mammalian cell culture and is unable to replicate in insect cell culture. The separated expression of Gn and Gc from two different genome segments resulted in an optimally safe and effective vaccine virus (Wichers Schreur et al., 2015. Vaccine 33: 1459-1464; Wichgers Schreur et al., 2015. Vaccine 33: 1459-1464).

The term "avirulent virus", as is used herein, refers to a live, infectious virus that is not pathogenic. An avirulent virus is preferably able to replicate its genome and to express genome-encoded proteins. Said virus is avirulent, for example, because it lacks expression of the NSs gene and does not spread from an infected cell.

The term "tumor-associated antigen", as used herein, is known in the art and refers to an antigen that is expressed by a specific tumor. Said antigen preferably is not expressed in normal cells, or at a lower level. Examples of a tumor-associated antigen are Cancer Antigen 125 (CA-125), which is a protein that is present on the surface of ovarian cancer cells and some other cancers, and in small amounts in normal tissue; Melanoma differentiation antigens (MDAs) such as pMEL17/gp100, tyrosinase, gp75/tyrosinase related protein (TRP)-1, dopachrome tautomerase/TRP-2, and MART-1/melan-A are ideal target antigens due to their preferential expression in melanocytes and melanoma cells (Hearing et al.,1992. Pigment Cell Res 5: 264-270); prostatic acid phosphatase which is highly expressed in prostate tumor cells; NY-ESO-1, which is expressed in some prostate tumor cells; MAGE-A3, which is expressed in melanoma and lung cancer cells and carcinoembryonic antigen, which is expressed in epithelial tumors, particularly colorectal cancers (Hance et al., 2005. Mutat Res 576: 132-154); Wilm's tumor protein associated with hematologic malignancies and a vast variety of solid tumors, including breast cancer, leukemia glioblastoma, ovarian cancer and embryonic malignancies of the kidney (Qi et al., 2015. Sci Rep 9: 8924).

The term tumor-associated antigen includes both a full length protein, as well as a part of a protein that is capable of inducing an immune response.

A preferred Phlebovirus-based vector according to the invention is a RVFV-based vector. As is indicated herein above, vaccines based on RVFV particles lacking NSs were shown to be remarkably immunogenic: A single vaccination with NSR expressing the Gn gene from the S segment provided sterile RVF immunity after a single vaccination of lambs (Oreshkova et al., 2013. PloS one 8:e77461; Kortekaas et al., 2014. Vaccine 32: 4901-8); A single vaccination with RVFV-4s particles lacking NSs was equally, if not more effective than NSR vaccination in lambs (Wichgers Schreur et al., 2015. Vaccine 33: 1459-1464). A single vaccination with NSR particles expressing the hemagglutinin of influenza virus protected mice from lethal influenza (Oreshkova et al., 2014. Vaccine 32: 5323-5329).

Apart from the induction of strong antibody responses, analyses of cellular immune responses demonstrated that NSR vaccination elicits Th1-balanced immune responses. A Th1-balanced immune response is particularly desirable for the control of intracellular pathogens (viruses, intracellular bacteria and parasites); and for controlling cancer. In support of the latter, vaccination with NSR particles expressing a single CD8-restricted epitope can control outgrowth of tumor cells in mice (see Example 2).

An avirulent, recombinant, non-spreading, genome replication-competent Phlebovirus-based vector expressing a tumor-associated antigen is preferably purified. Methods for purifying Phlebovirus particles, especially RVFV particles, are known in the art. For example, purification of Phlebovirus particles may be accomplished by harvesting culture medium of infected cells and clarifying medium by centrifugation, followed by concentration via ultrafiltration and concentration using hollow fibre membrane cartridges. Further purification can be performed by gel filtration using e.g. Sepharose. Preferably, the virus is found in the void volume and DNA and protein contaminants are retarted. Further purification can be performed by anion exchange chromatography, where DNA and negatively charged proteins are bound to the column. Finally, final concentration can be performed via a second ultrafiltration/diafiltration step using hollow-fibre membrane cartridges.

The invention further provides a Phlebovirus-based vector according to the invention for use in a method of treating an individual suffering from a tumor.

The term "tumor", as used herein, refers to a disease that is characterized by uncontrolled growth of cells. This uncontrolled growth may be accompanied by intrusion into and destruction of surrounding tissue and possibly spread of tumor cells to other locations in the body. The term tumor includes cancers derived from epithelial cells, termed carcinomas, such as breast, ovarian, prostate, lung, pancreas, and colon carcinomas; cancers arising from connective tissue termed sarcomas; cancers that arise from cells that make blood, termed lymphoma and leukemia; germ cell tumor; melanomas; and cancers derived from immature precursor cells or embryonic tissue, termed blastomas.

The tumor may be selected from the group consisting of acute lymphoblastic leukemia, acute myeloid leukemia, adrenocortical carcinoma, colorectal cancer, appendix cancer, astrocytoma, atypical teratoid/rhabdoid tumor, basal cell carcinoma, bile duct cancer, bladder cancer, brain stem glioma, breast cancer, Burkitt lymphoma, cerebellar astrocytoma, lymphocytic leukemia, chronic myelogenous leukemia, craniopharyngioma, endometrial cancer, ependymoblastoma, ependymoma, esophageal cancer, extracranial germ cell tumor, extragonadal germ cell tumor, gallbladder cancer, gastrointestinal cancer, gastrointestinal stromal tumor, gestational trophoblastic tumor, glioblastoma, hairy cell leukemia, head and neck cancer, hepatocellular cancer, Hodgkin lymphoma, hypopharyngeal cancer, hypothalamic and visual pathway glioma, intraocular melanoma, kaposi sarcoma, laryngeal cancer, leukemia, meduloblastoma, meduloepithelioma, melanoma, merkel cell carcinoma, mesothelioma, mouth cancer, multiple endocrine neoplasia syndrome, multiple myeloma, myeloma, mycosis fungoides, nasal cavity and paranasal sinus cancer, nasopharyngeal cancer, neuroblastoma, non-hodgkin lymphoma, non-small cell lung cancer, oral cancer, oropharyngeal cancer, osteosarcoma, ovarian cancer, ovarian epithelial cancer, ovarian germ cell tumor, ovarian low malignant potential tumor, pancreatic cancer, papillomatosis, paranasal sinus and nasal cavity cancer, parathyroid cancer, penile cancer, pharyngeal cancer, pheochromocytoma, pituitary tumor, pleuropulmonary blastoma, primary central nervous system lymphoma, prostate cancer, rectal cancer, renal cell cancer, retinoblastoma, rhabdomyosarcoma, salivary gland cancer, sezary syndrome, small cell lung cancer, small intestine cancer, soft tissue sarcoma, squamous cell carcinoma, squamous neck cancer, testicular cancer, throat cancer, thymic carcinoma, thymoma, thyroid cancer, urethral cancer, uterine sarcoma, vaginal cancer, vulvar cancer, Waldenstrom macroglobulinemia, and Wilms tumor.

The invention further provides a method of presenting at least one antigenic peptide at the surface of an antigen-presenting cell (APC), the method comprising a) infecting said APC with a Phlebovirus-based vector according to the invention; b) allowing the APC to present said antigenic peptide.

The term "antigen presenting cell", as used herein, refers to immunocompetent cells that mediate cellular immune responses by processing and presenting antigenic peptides to the T-cell receptor, including macrophages, dendritic cells, Langerhans cells, and B-lymphocytes, and precursors of these cells. The antigen is degraded into peptides which are then displayed at the surface of the cell by a class I histocompatibility molecule. Dendritic cells and macrophages may also cross-present antigens after phagocytosis of an apoptotic Phlebovirus vector-infected cells, resulting in both MHC-I and MHC-II presentation Finally, both cell types may present intact antigen directly to B cells.

A preferred antigen presenting cell is a dendritic cell (DC). DCs reside in tissues that are often exposed to the external environment such as the skin and represent the first line of defense against invading pathogenic organisms. Derived from hematopoietic progenitor cells, DCs only differentiate and mature after contact with an antigen. DCs express costimulatory molecules, such as CD80 (B7-1), CD86 (B7-2), and CD40, that are important for activation of primary cellular immune responses (Banchereau et al., 2000. Ann Rev Immunol 18: 767-811; Mellman and Steinman, 2001. Cell 106: 255-258).

Said antigen presenting cell is preferably activated to mature into a fully functional dendritic cell, prior to or after contacting said APC with a Phlebovirus particle, whereby the fully functional dendritic cell expresses costimulatory molecules, such as CD80 (B7-1), CD86 (B7-2), and CD40.

The invention further provides a method of ameliorating and/or treating an individual suffering from a tumor, the method comprising administering an effective amount of a Phlebovirus-based vector according to the invention to said individual to stimulate an immune response in the individual against a tumor-associated antigen, thereby ameliorating and/or treating said individual.

The term "effective amount", as used herein, means an amount of a Phlebovirus-based vector according to the invention, that produces an effect on the cancer to be treated. An effective amount is between 10E3 and 10E12 particles, preferably between 10E7 and 10E10 particles. The amount of particles preferably refers to the amount of live, infectious particles and may be determined by, for example, plaque assays to determine the number of plaque forming units (pfu), and/or assays to determine the 50% Tissue Culture Infective Dose (TCID50), which indicates the amount of virus required to infect 50% of infected cells *in vitro.* The amount of particles that is administered, the dosage unit, preferably is between 10E3 and 10E12 TCID50 particles.

A Phlebovirus-based vector according to the invention preferably is administered to an individual suffering from a tumor by parenteral administration, including intramuscular, subcutaneous, intraperitoneal, preferably intramuscularly, more preferably intradermal. A typical treatment schedule or dosing regimen comprises parenteral administration, preferably intradermal injection and/or intramuscular injection, of one dosage unit.

A Phlebovirus-based vector according to the invention may be administered together with immune-stimulants. Said immune-stimulants may comprise recombinant, synthetic and natural preparations. Preferred immune-stimulants are cytokines, including granulocyte colony-stimulating factor (G-CSF), interleukins such as IL-2, IL-7, and/or IL-12, and interferons, but may also include imiquimod (3-(2-methylpropyl)-3,5,8-triazatricyclo[7.4.0.02,6]trideca-1(9),2(6),4, 7,10,12-hexaen-7-amine), synthetic cytosine phosphate-guanosine (CpG), glucans, and cellular membrane fractions from bacteria such as *Mycobacterium* spp., *Corynebacterium parvum, C. granulosum, Bordetella pertussis* and *Neisseria meningitides,* and/or the isolated membrane-bound product N-acetyl muramyl-L-alanyl-D-isoglutamine.

A Phlebovirus-based vector according to the invention is preferably administered without an additional immune-stimulant. An advantage of a Phlebovirus-based vector according to the invention is that it provides a good immune response in the absence of an adjuvans.

A preferred method comprises providing *ex vivo* human antigen processing cells (APCs), infecting said APCs with a Phlebovirus-based vector according to the invention, and administering said infected APCs to said individual.

The amount of Phlebovirus particles that is used to infect, ex vivo, APCs is between 10 and 10E4 particles per cell, whereby the amount of particles preferably refers to the amount of live, infectious particles. The multiplicity of infection (MOI) therefore preferably is between 10 and 10.000 TCID50 particles per cell.

Said APC preferably is a dendritic cell or a precursor of a dendritic cell. Said dendritic cell or precursor thereof is preferably matured prior to administering said infected APCs to said individual. Maturation of DC can be accomplished by the application of cytokine cocktails which include cytokines such as Flt-3 ligand, M-CSF, GM-CSF, and/or IL-4. Maturation can be achieved by infecting the APC with Phlebovirus-based vectors according to the invention or by infecting already mature APCs with Phlebovirus-based vectors according to the invention.

At least two subtypes of DC have been recognized, myeloid DCs (mDCs) and plasmacytoid DCs (pDCs).

Said DC preferably is a mDC. Precursors of mDC are CD34+ progenitor cells and monocytes. Monocytes can be isolated by plating of peripheral blood mononuclear cells in a tissue culture flask, thereby permitting adherence of especially monocytes. If needed, monocytes may be purified based on CD14-expression, for example by using a CliniMACS® system (Miltenyi). Incubation of monocytes in the presence of GM-CSF (5-100 ng/ml) and interleukin 4 (IL-4) (2-50 ng/ml), with or without Tumor Necrosis factor alpha (TNF-alpha), will result in the differentiation of monocytes into immature dendritic cells.

As an alternative, the number of circulating DCs in a patient may be increased by 10-30 fold using Flt3-ligand (Fong et al., 2001. PNAS USA 98: 8809-8814). These cells can be harvested with a leukapheresis procedure and used for infection with a Phlebovirus according to the invention.

In one embodiment, said APC is a human dendritic cell line, which is able to stimulate T-cells of the immune system to attack cancer cells, thereby aiming to cure the patient from cancer. It is preferred to use DC from a readily available and unlimited source, such as a cell line, as the availability of DC precursors is often limited, and the preparation of individual vaccines is labor-intensive. It has been described that leukemia-derived cell lines are able to differentiate into functional DC (Santegoeds et al., 2008. J Leukoc Biol 84: 1364-73), allowing their use in methods of the invention.

It is preferred that said APCs are infected by a Phlebovirus-based vector after administering said vector to said individual. The administration of a Phlebovirus-based vector is likely to result in activation of bystander DCs, thereby enhancing an anti-tumor-directed immunological response.

In another preferred embodiment, the APCs are isolated, preferably as DC precursor cells, from the tumor patient prior to infection with the Phlebovirus-based vector. The use of autologous APC is an optimally safe approach and excludes the risk of incompatibility.

When APCs are infected with a Phlebovirus-based vector *in vitro,* it is preferred that the APCs are differentiated into fully functional dendritic cells, prior to infecting said APCs with a Phlebovirus particle. Mature APCs are immunogenic in that they express cell surface molecules important for T cell activation. Maturation of APCs, especially DCs, is accomplished by cultivating cells in the presence of a pro-inflammatory cytokine cocktail including, for example, TNF-α, IL-Lβ, IL-6 (CellGenix) and 1 µg/ml PGE2 (Pfizer). Maturation of DCs is associated with ligation of the co-stimulatory receptor CD40 (also known as TNFRSF5).

Infected APCs either directly or indirectly activate T cells. Indirect activation through bystander DCs may amplify the immune response. Infected DCs produce and secrete cytokines, which lead to the activation of bystander DCs. In addition, when infected DCs go into apoptosis before reaching T-cell areas of lymphoid organs, the cells can be phagocytosed by activated bystander DCs. This process results in so-called cross-presentation of antigenic peptides. When the viral vector encodes a tumor-associated antigen, cross-presentation will result in optimal presentation of tumor-associated antigen-derived MHC-I and MHC-II restricted peptides. The cross-presenting DCs will migrate to lymphoid organs and interact with corresponding T cells, resulting in their activation. Without being bound by theory, the strong immune response mediated by infection of APCs with Phlebovirus particles, may at least in part be explained by activation of bystander DCs. The transient downregulation of CD83 surface expression on infected APCs seems to support a role for bystander DCs in the immune response elicited by an avirulent Phlebovirus-based vector encoding a tumor-associated antigen.

### Figure legends

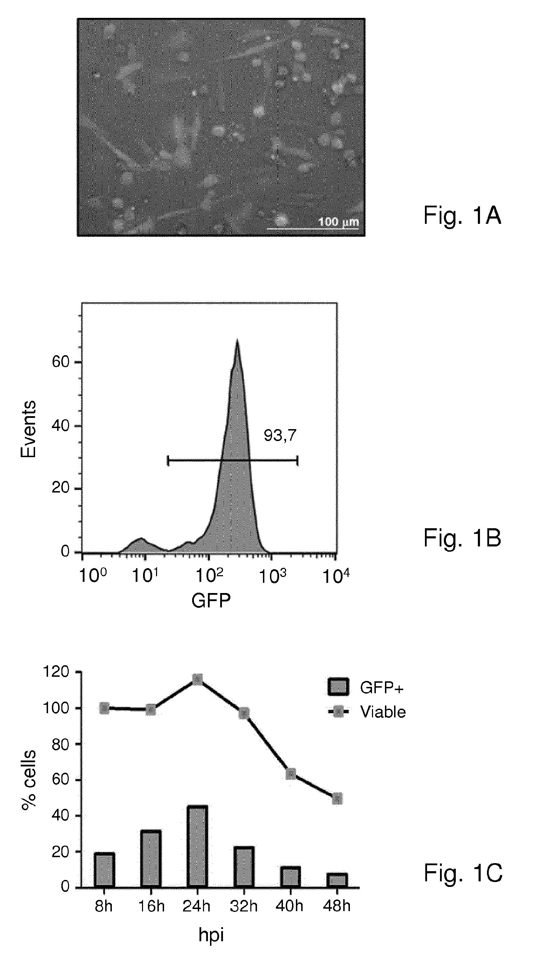

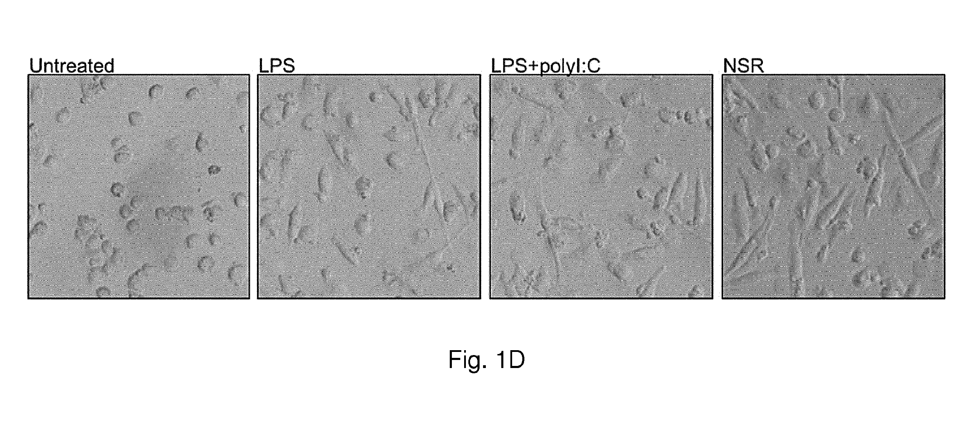

Figure 1.- Infection of DCs by NSR results in morphological changes characteristic of an activated state. (A) Replication of NSR genome segments results in expression of viral proteins, evidenced by GFP expression. DCs were incubated with NSR for 24 h. (B) Maximal infection efficiency of NSR on DCs. (C) Infection progress and changes in viability of infected DCs in time. Cells were infected with NSR, harvested at the indicated time points and analysed by flow cytometry for GFP expression (bars) and viability (line) after staining with 7AAD. Viability of the cells was calculated relative to that at 8 hpi, which was set at 100%. The data shown are averages from two experiments, from two different donors (D) Morphology of unstimulated DCs (left panel), cells stimulated with LPS or a combination of LPS and poly I:C (middle panels) or infected with NSR (right panel) at 24 h after treatment.

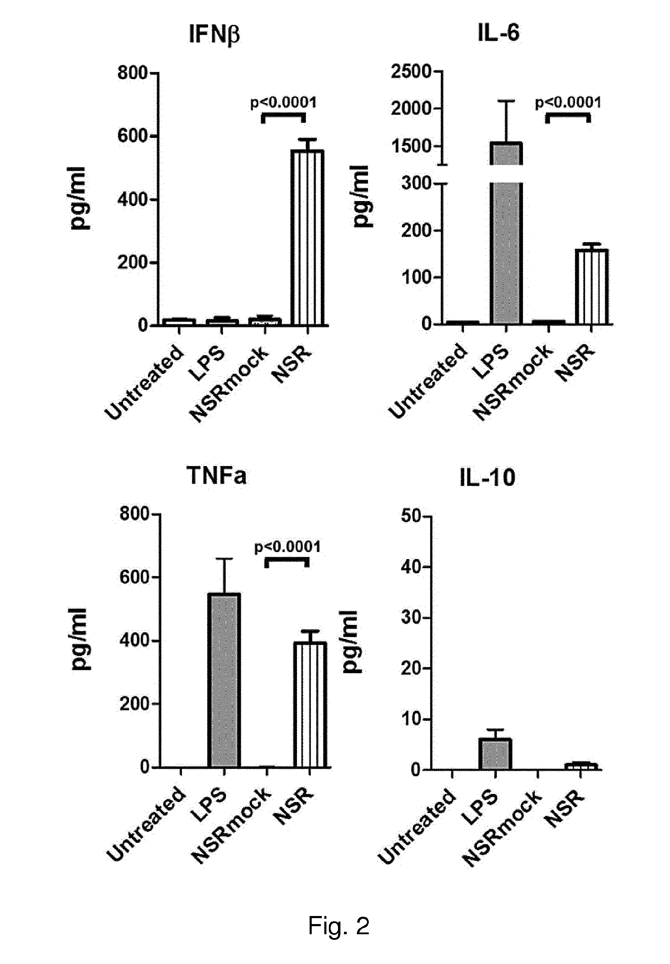

Figure 2 - Cytokine secretion by NSR-infected DCs. Supernatants of infected or control-treated DCs were analysed with multiplex assay at 24 hpi. Bars represent the mean cytokine concentrations ± SD of triplicates with cells from one donor. Statistical significance between infected (NSR) and mock-infected (NSRmock) conditions is indicated (Student's t test).

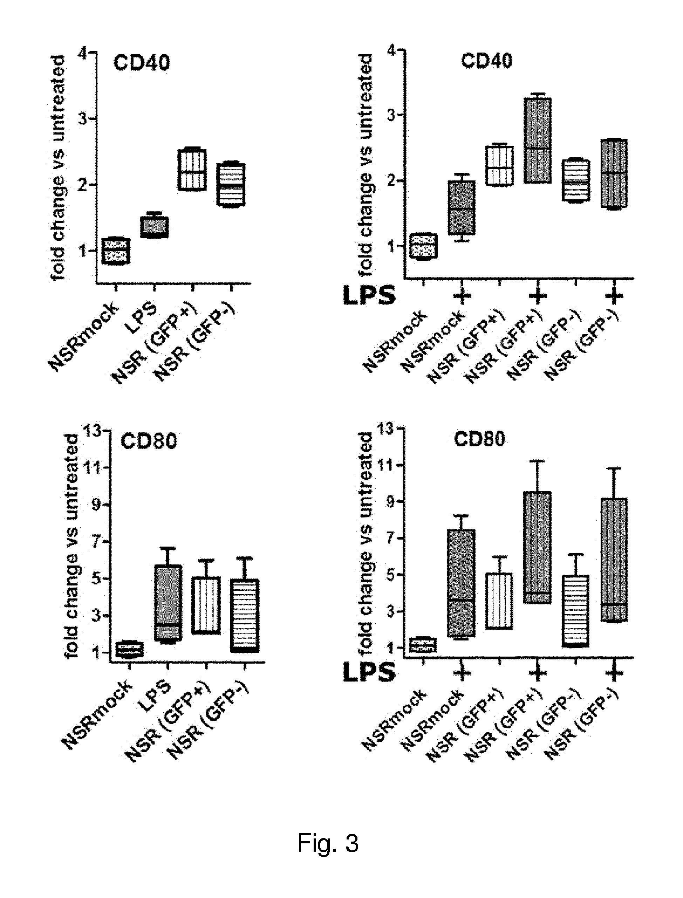

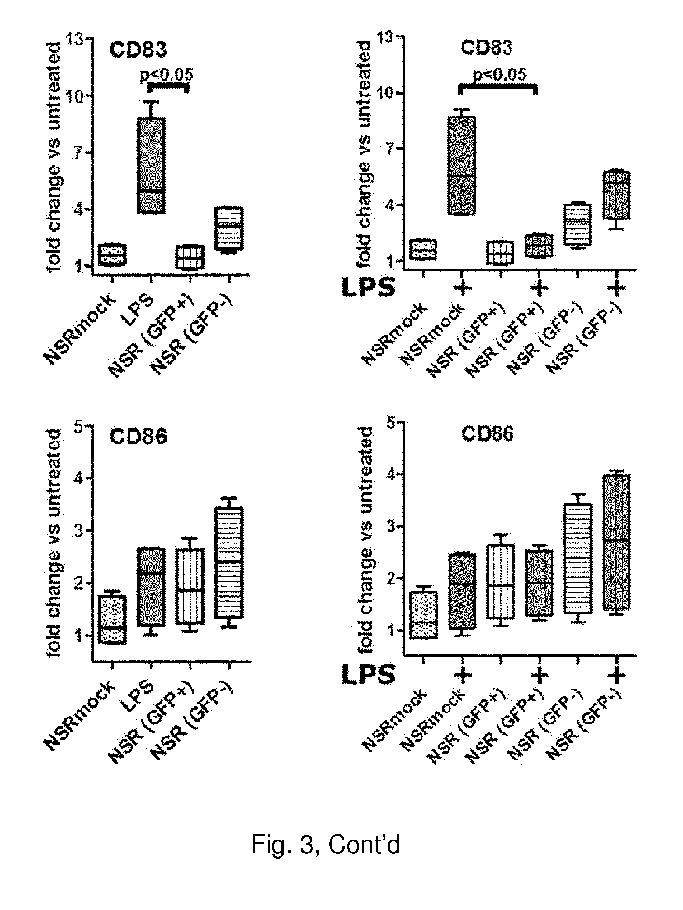

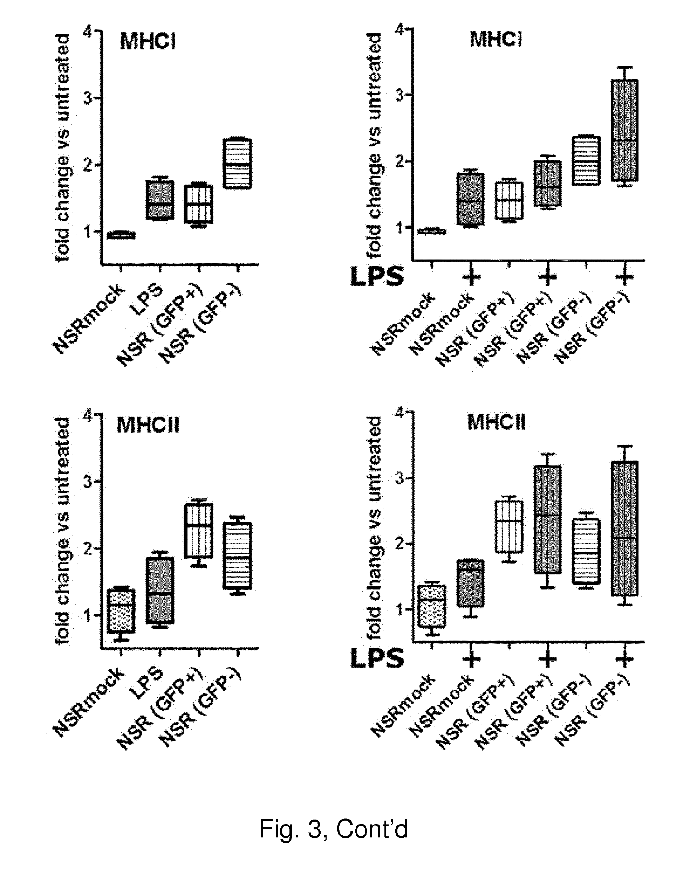

Figure 3- Surface expression of CD40, CD80, CD83, CD86, MHC-I and MHC-II on DCs at 24 h after NSR infection as measured by flow cytometry. Immature DCs were infected with NSR, incubated with the controls NSRmock or NSRinact, or stimulated with LPS. All surface markers in NSR- infected cells are upregulated except CD83. Incubation of DCs with NSR+LPS resulted in an additional increase in expression of the surface markers (right panels). However, LPS is not able to rescue expression of CD83 in NSR-infected cells. The left panels depict summarized data from 4 independent experiments performed with cells from 3 donors. Data in the right panels further elaborates on the data in the left panel, comparing single NSR treatments with combined LPS and NSR treatments. The box plots depict median surface expression of the different markers relative to untreated cells. Statistically significant differences of CD83 expression levels are indicated (paired T test).

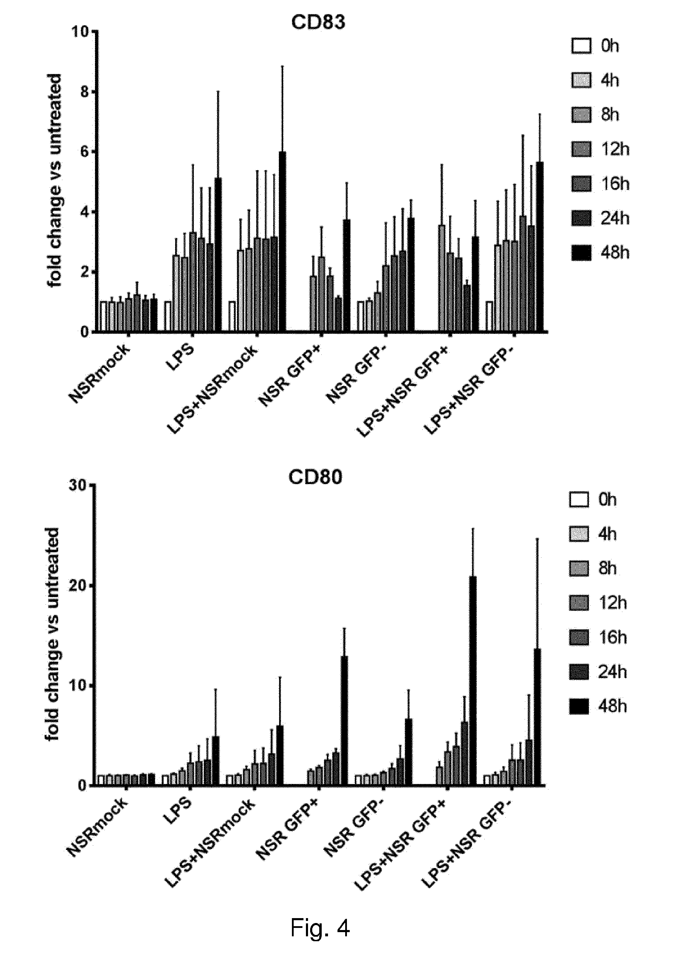

Figure 4 - Analysis of CD83 and CD80 surface expression in time. DCs were treated with NSRmock, NSR, LPS, LPS+NSRmock or LPS+NSR and were harvested at 0, 4, 8,12, 16, 24 and 48 h after treatment. Surface expression of CD83 (upper panel) and CD80 (lower panel) were measured by flow cytometry. Time points are depicted with different grey shades and the shade code is shown at the right. IC - isotype control. Shown are summarized data from three independent experiments with cells from three different donors. Bars represent means + SD of the fold change of MFI relative to untreated cells.

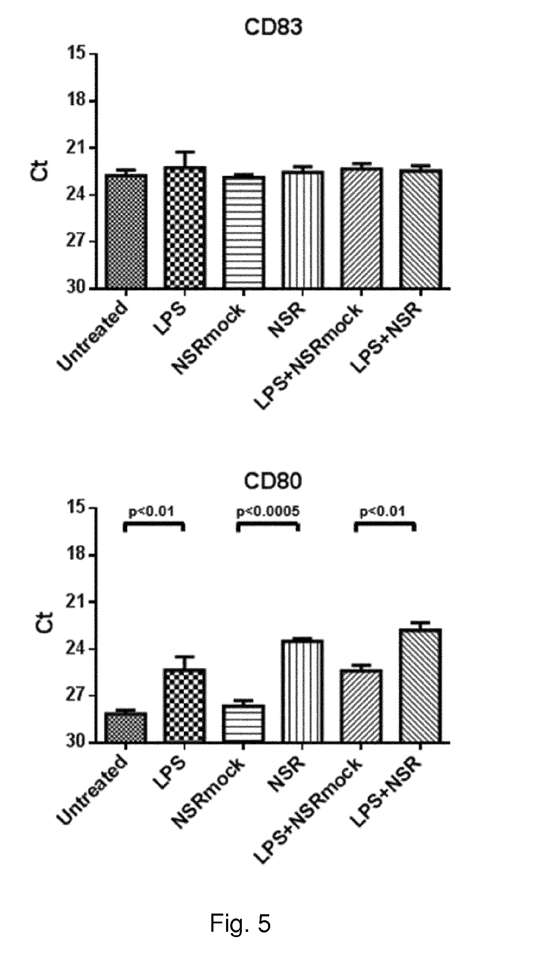

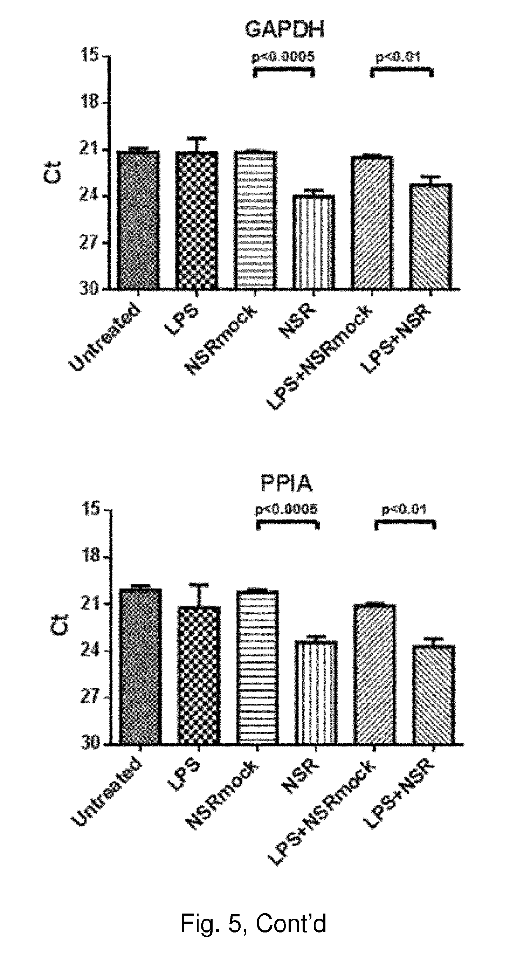

Figure 5 - Quantities of CD83, CD80, GAPDH and PPIA mRNAs in cell lysates at 24 h after different treatments, analysed with qPCR. Bars represent average Ct values from three repetitions with standard deviation. Cells were derived from one donor, but similar results were obtained from cells from two further donors. Relevant statistical significance is indicated (Student's t test).

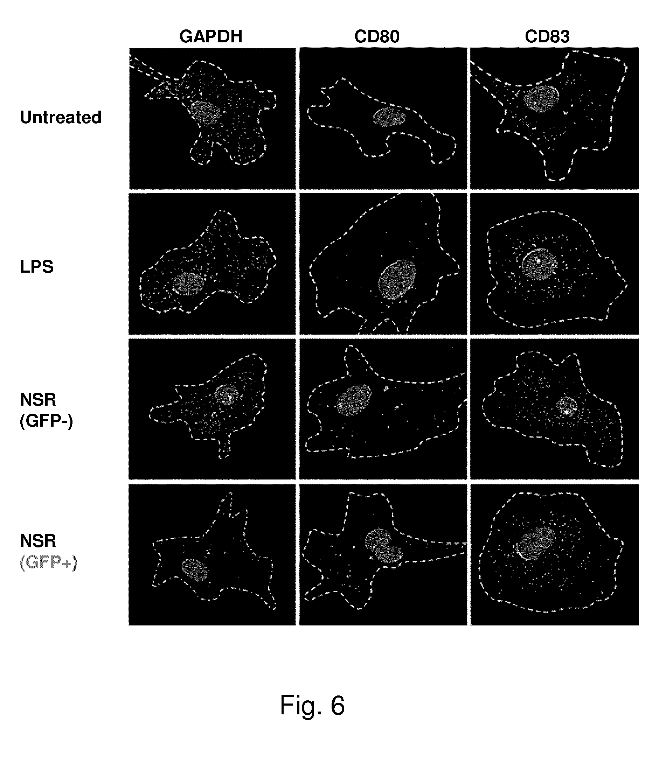

Figure 6 - Visualization of GAPDH, CD80 and CD83 mRNAs with fluorescence in situ hybridisation (FISH). DCs were stimulated for 24 h with LPS, NSR or left untreated and then fixed and subjected to FISH. Shown are representative cells of each treatment condition from two independent experiment with cells from two donors.

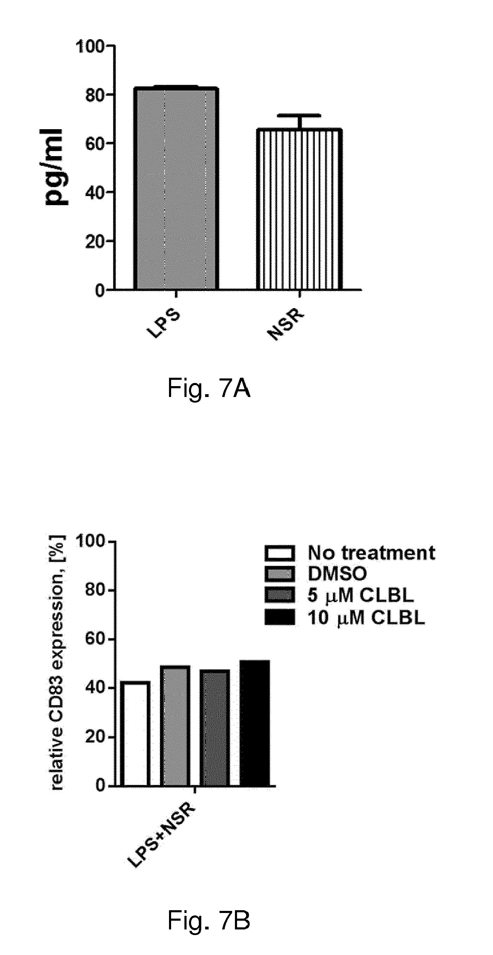

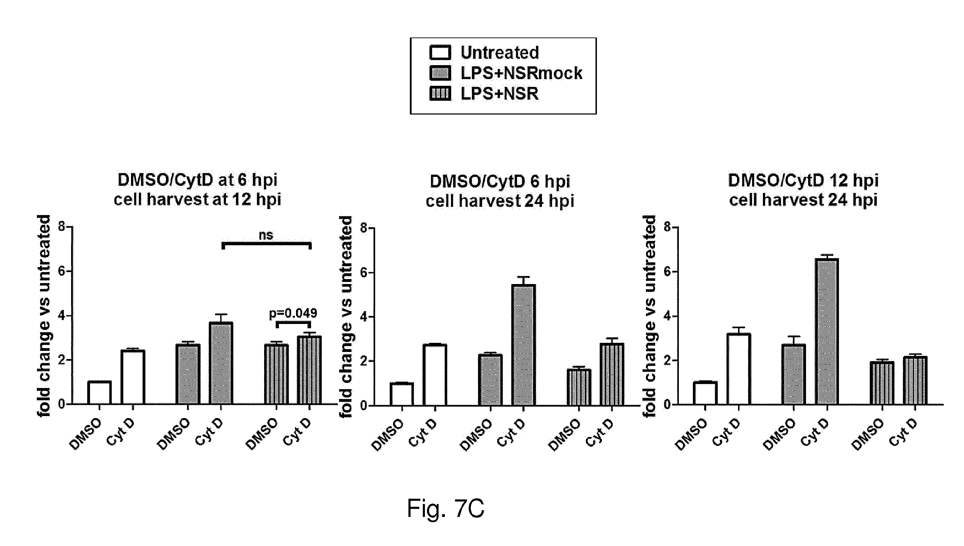

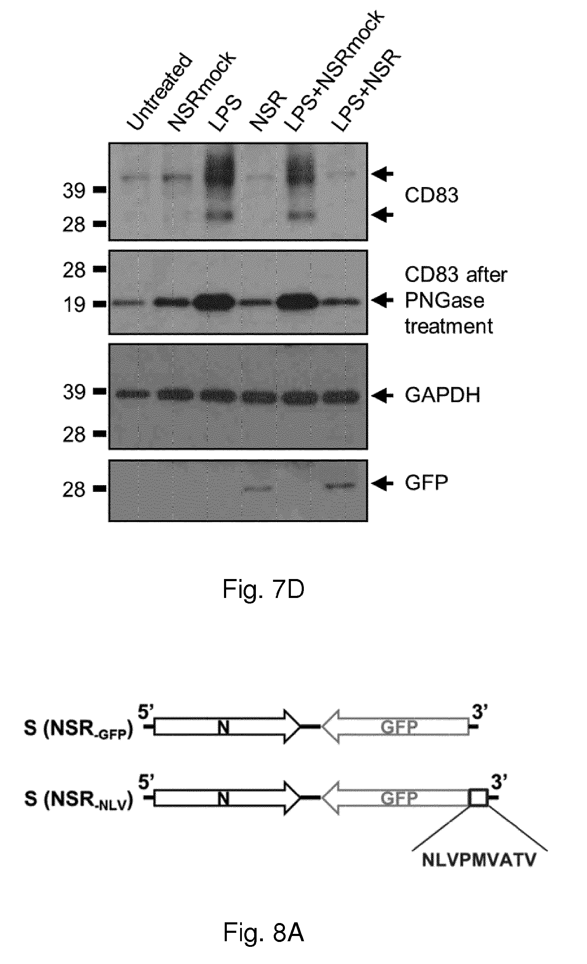

Figure 7 - Effect of NSR infection on intracellular and extracellular CD83 levels. (A) Levels of CD83 in supernatants were determined by ELISA of supernatants from cells harvested 24 h after stimulation with LPS or NSR. The depicted data represent one of two independent experiments performed with cells from two different donors. (B) Flow cytometry analysis of CD83 surface expression after inhibition of the proteasome. DCs were stimulated with LPS+NSRmock or LPS+NSR for 8 h and then clasto Lactacystin β-lactone (CLBL) was added at two final concentrations. Control cells were left untreated or treated with DMSO. Cells were analysed at 24 hpi for CD83 expression. Bars represent percentage of mean fluorescent intensity in LPS+NSR infected cells, relative to LPS+NSRmock. The treatment conditions are depicted in the legend. Averaged values of two experiments from 1 donor are shown. (C) Inhibition of endocytosis. DCs were stimulated with LPS+NSRmock, LPS+NSR or left unstimulated. Cytochalasin D (Cyt D) or the solvent DMSO were subsequently added at different time points. The moments of adding Cyt D/DMSO and harvesting of cells are indicated above each graph. Bars represent average fold change of the median fluorescence intensity relative to unstimulated cells, treated with DMSO. Error bars represent standard deviation. Averaged values of three experiments with cells from one donor are depicted. (D) Detection of CD83 in cell lysates by Western blot at 24 hpi. The different treatments are shown above the top panel and the probed proteins are depicted at the right. Molecular weight protein standard is shown at the left. GAPDH was used as a loading control and GFP was used to confirm NSR infection.

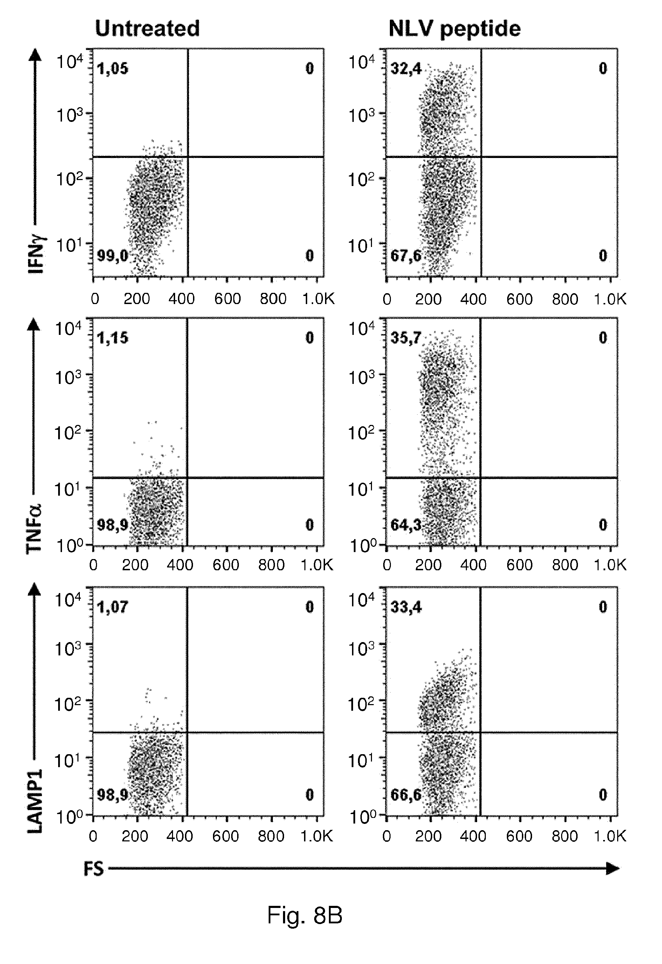

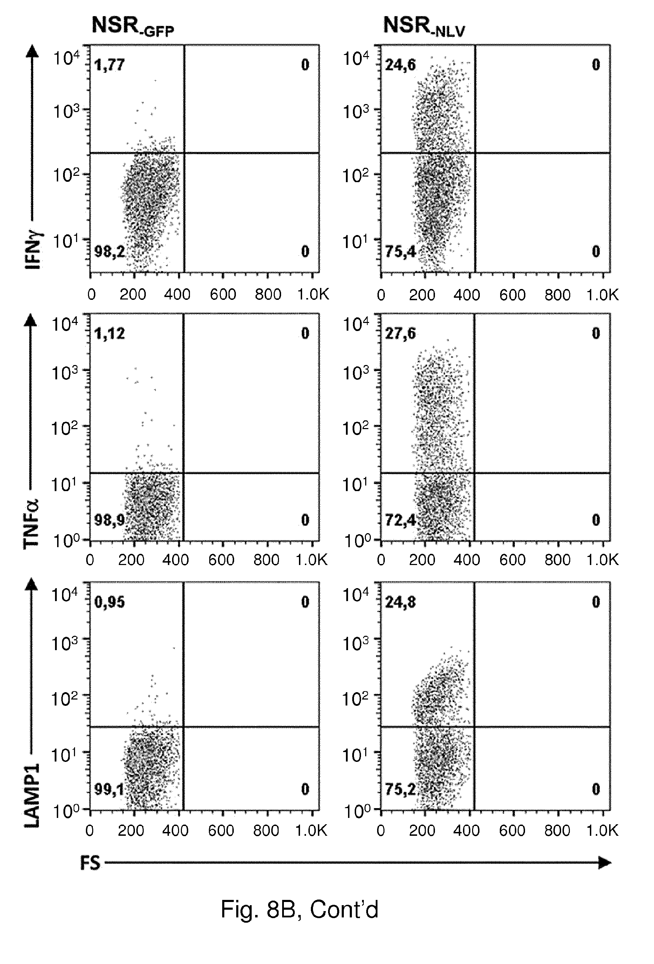

Figure 8 - A) Schematic representation of the NSR S segment encoding GFP (top) or C-terminally fused pp65495-503 epitope (bottom) in anti-genomic orientation. B) NSR-infected human DCs activate antigen-specific CD8+ T-cells. HLA A2+ DCs were infected with NSR-GFP or NSR-NLV and were cultured O/N. As negative control, DCs were left untreated; as positive control, DCs were loaded with synthetic peptide pp65495-503. A2-restricted CD8+ T cell clone, specific for the pp65495-503 epitope was added for 4.5h in the presence of brefeldin A, and analyzed for activation. The expression of IFN-γ, TNF-α and LAMP-1 of CD8/CD3 positive population is depicted. All three parameters are induced specifically in NSR-NLV infected cells and not in NSR-GFP infected cells.

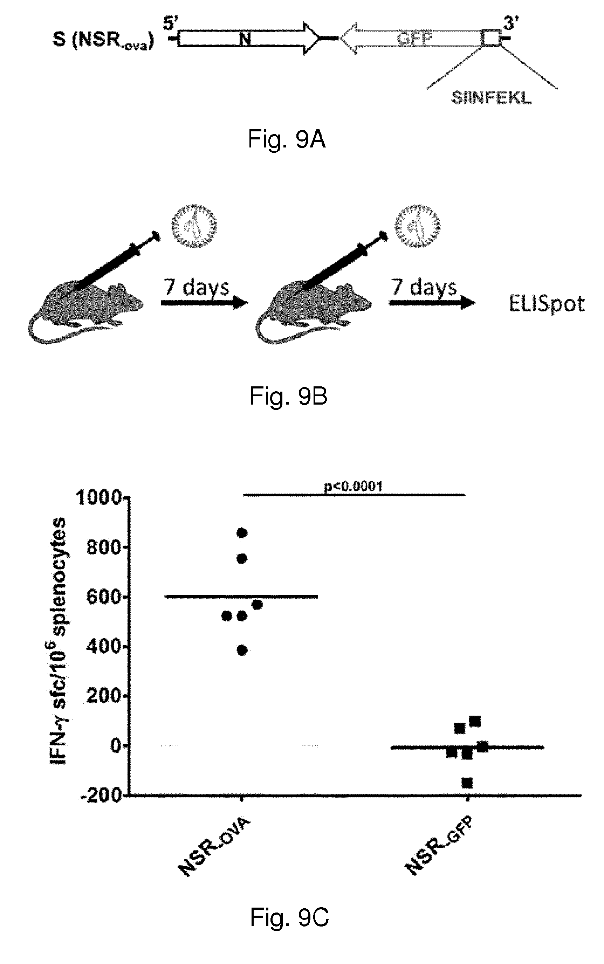

Figure 9 - A) Schematic representation of the NSR S segment encoding the OVA257-264 peptide fused to GFP in anti-genomic orientation. B) Representation of the vaccination regimens with NSR-OVA or control NSR-GFP vaccine. C) ELISpot assay showing IFN-γ responses of splenocytes collected from mice that were vaccinated with either NSR-GFP or NSR-OVA (n=6). Symbols represent individual counts of IFN-γ spot forming cells. Statistical significance is indicated (Student's t-test).

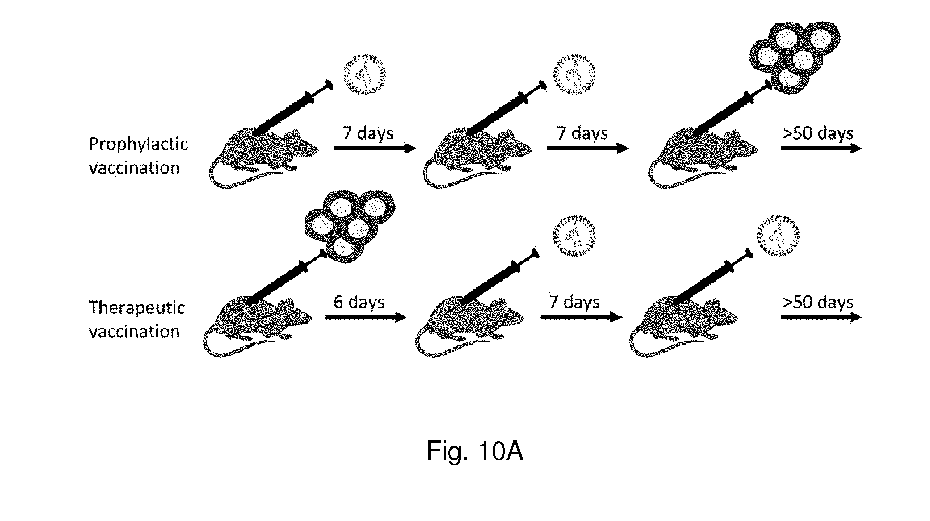

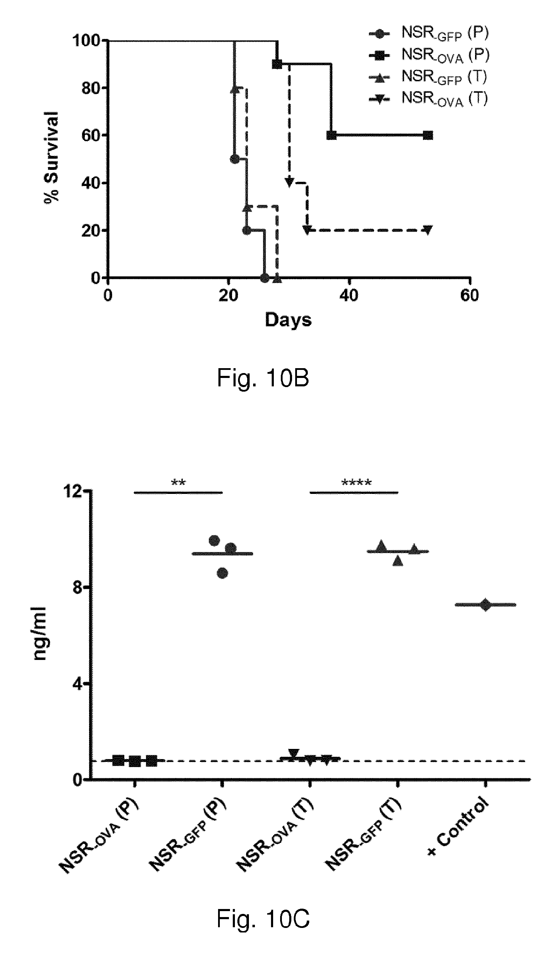

Figure 10 - A) Representation of the prophylactic and therapeutic vaccination regimens with NSR-OVA or control NSR-GFP vaccine. The priming therapeutic vaccination was applied when more than 50% of E.G7-OVA-inoculated mice had palpable tumors. B) Survival curve of mice prophylactically (P, solid lines) or therapeutically (T, interrupted lines) vaccinated with NSR-GFP or NSR-OVA. C) Detection of OVA in supernatants of cultured E.G7-OVA cells by ELISA. Cells were re-cultured for 7 days after being collected from mice (n=3/group) at the moment of euthanazation. Statistical significance is denoted (Student's t-test with Welch's correction for unequal variances). E.G7-OVA cells that were used for inoculation of the mice were used to obtain a positive control sample (+ Control). The animal experiment was conducted in accordance with the Dutch Law on Animal Experiments (Wod, ID number BWBR0003081) and approved by the Animal Ethics Committee of the Central Veterinary Institute (Permit Number: 2014101.c).

### Examples

### Example 1

### Materials and methods

Cells. Peripheral blood mononuclear cells (PBMCs) were isolated from blood of healthy donors by ficoll isopaque density gradient centrifugation (GE Healthcare Bio-Sciences AB) and frozen until use. PBMCs were used as a source of monocyte-derived dendritic cells (MoDCs). Immature DCs (iDCs) were cultured as previously described (Flinsenberg et al., 2012. Blood 120: 5163-5172). Briefly, PBMCs were seeded in standard culture plates in X-VIVO-15 medium with gentamycin (Lonza), supplemented with 2% heat-inactivated and 0.2 µm-filtered FCS (Bodinco). Cells were allowed to adhere for 1 h. Non-adherent cells were subsequently removed by washing with PBS and adherent cells were cultured in serum-free X-VIVO-15, supplemented with 450 U/mL GM-CSF and 300 U/mL IL-4 (Milteny). Medium was refreshed on the second day of incubation. On day 5, cells were stimulated with LPS, NSR or NSR controls (NSRmock and NSRinact) diluted in RPMI 1640 with HEPES and glutamine (Gibco), supplemented with 10% FCS. As a control for negative stimulation, medium was used. The stimulation conditions were selected for optimal infectivity of NSR. DCs were harvested at different time points after stimulation as indicated in the results section, by replacing the growth medium with cold PBS, followed by shaking (450 rpm) of the culture plates for 1 h at 4°C to detach cells. Further detachment was achieved by careful pipetting up and down and the loose cells were harvested for further analysis.

### Generation of Nonspreading RVFV (NSR) and control inocula

**(NSRmock and NSRinact).** NSR particles were generated as previously described (Oreshkova et al., 2013. PloS one 8: e77461). Briefly, NSR replicon cell lines were transfected with a plasmid encoding the viral surface glycoproteins Gn and Gc. NSR containing supernatants were harvested the next day and cleared from cell debris by centrifugation at 4500 xg for 15 min. Concentration of NSR particles was achieved by ultracentrifugation at 64,000 xg for 2.5 h, followed by resuspension in Opti-MEM (Invitrogen), supplemented with 0.2% heat-inactivated FCS. Particles were stored at -80°C until use. For generation of NSRmock, an similar procedure was used, but the replicon cells were transfected with a plasmid that encodes only the Gc protein. Supernatants, harvested after this transfection contain the same media, transfection reagents and cellular metabolism products, but lack infectious NSR particles. NSRinact was prepared by inactivation of NSR with UV-light (254 nm) for 20 minutes. The absence of infectious particles in both NSRmock and NSRinact control preparations was confirmed by titration on BHK21 cells.

**Flow cytometry.** The cell-surface phenotypes of unstimulated and stimulated DCs were analysed by flow cytometry using human-specific mAbs: anti-CD40 (clone 5C3) and anti-CD83 (clone HB15e) (eBioscience); anti-CD80 (clone L307.4), anti-CD86 (clone IT2.2) and anti-CD11c (clone B-ly6) (BD); anti-HLA-A,B,C (clone W6/32) and anti-HLA-DR (clone L243) (BioLegend), together with the respective isotype controls. Optimal concentrations of the antibodies were determined prior to flow cytometry assays. The DC population was selected by gating on cells that were double positive for CD11c and MHC-II. Median fluorescence intensity (MFI) was used as a measure for expression of the analysed molecules.

**Cytokine assay.** PBMCs were seeded in 48-well culture plates and, after an initial adherence step for 1 h as described above, were washed vigorously with PBS so that only adhering cells were retained. After 5 days of differentiation, DCs were stimulated with LPS, NSR, NSRmock or medium only and after 24h supernatants were harvested, pre-cleared at 6,000 rpm for 10 min and stored at -80°C until use. Cytokine concentrations were determined using a multiplex assay (eBioscience), according to the manufacturers' instructions using the Luminex 200 system.

**Polyacrylamide gel electrophoresis (PAGE) and Western blotting.** DCs were harvested 24 h after stimulation with different stimulae, counted and brought to equal concentrations in Pierce IP lysis buffer (Thermo Scientific), supplemented with protease inhibitors (Roche). Samples containing 50,000 cells were either directly denatured in standard Laemmli sample buffer or first pre-treated with PNGase F according to the manufacturers' instructions (BioLabs^{®} Inc.). Proteins present in cell lysates were separated by sodium dodecyl sulfate polyacrylamide gel electrophoresis (SDS-PAGE), followed by Western blotting as previously described (Oreshkova et al., 2014. Vaccine 32: 5323-5329). Rat monoclonal anti-human CD83 antibody (clone 1G11, Enzo Life Sciences) and corresponding secondary horseradish peroxidase (HRP)-conjugated antibody were used to detect CD83. The blot with samples without PNGase treatment was subsequently stripped and re-stained with mouse monoclonal anti-human GAPDH (clone 0411) and anti-GFP (clone B-2) antibodies (Santa Cruz) and corresponding secondary HRP-conjugated antibodies.

**Single-molecule RNA fluorescence in situ hybridization.** DCs were cultured on a CultureWell™ 16 Chambered Coverglass (C-37000, Grace Biolabs) and stimulated as indicated. 24 h post stimulation cells were fixed with 4% paraformaldehyde (10 min) and permeabilized with 70% ethanol (>1 h at 4°C). Individual intracellular GAPDH, CD80 and CD83 mRNAs were subsequently visualized using human specific CD80, CD83 and GAPDH cDNA probes labelled with QUASAR 570® (Biosearch Technologies) according the Stellaris® FISH method using the protocol for adherent cells (Raj et al., 2008. Nat Methods 5: 877-879). The oligonucleotides for GAPDH were predesigned by Biosearch Technologies (SMF-2026-1) whereas the oligonucleotides for CD80 and CD83 were designed using the online Stellaris probe design software. Images were generated using an Axioskop 40 (Zeiss) fluorescent microscope with a 1.28 NA 100× oil objective and an AxioCam MRm camera. Raw images were deconvolved and analysed using Huygens software (Scientific Volume Imaging, Hilversum, The Netherlands).

The CD80 probes comprised, from 5'to 3':

gagttcactcagtacttgtc, tctgttactttacagagggt,atcttcagagaggcgacatt,
gtggatttagtttcacagct, gggctgatgacaatccaatt, aaagccaacaatttggaccc,
cttcagatgcttagggtcaa, gaggtatggacacttggatg, gaagtgagaaagaccagcca,
tgccagctcttcaacagaaa, caaagatggtccggttcttg, cgcttgaaagcgtctttttc,
tgtagggaagtcagctttga, tggaaaacctccagaggttg, ctgttgtgttgatggcattt,
agcatagagctcagtttcag, tgaaatccagtttgctgcta, gagacacatgaagctgtggt,
tccagttgaaggtctgattc, ggaaaatgctcttgcttggt, gtcaggcagcatatcacaaa,
ctgcggacactgttatacag, ggaccttcagatcttttcag, aagttcccagaagaggtcaa,
caaggtggggtaatcttgtc, agcacctaagagcagatacg, agacacttcctgcaaagcaa,
cttgatcaaggtcaccagag, gtattctctaaagtcccttc, gggaaagagcaccagagtta,
aggccacagtcaaactgata, aaattctacttccagcagca, tctctgaagttgacctgtta,
ctatgcattacattgggctt, tcctaaagatgttcatgcca, tcgtcttacatgtcagagga,
cttgactactgctttgacgt, ttaagtctttggactatccc, ccctatggaaagttactacc,
ccatcttagggatctaagga, acccagaaataccaaggaga, gctggctctaaaggctttaa,
tagtgaggtagctaaagcca, aggagcaaggtttgtgaagc, ctgccctacactgagaatat,
ggaaacactgctagtacctt, gctgacaaagtatctgctgt, caactttgtttcttccctta.

The CD83 probes comprised, from 5'to 3':

ctcaggagcagaagctggag, caggagaccgtgtagggaac, accaccctccaataacttga,
tttgccccttctgatgatag, attgggggcgtcgaaagaac, ctgcagagtgcacctgtatg,

aagtctcttctttacgctgt, acaatctccgctctgtattt, ctgggaagatactctgtagc,
tccatgccagctttagaaaa, cactagccctaaatgcttat, ataccagttctgtcttgtga,
agaacctgcagaaatcctgc, cagtgtaacagacaggcaca, ttcagcgtaggctcattctt,
tgaaggacttcacaggatgc, cttccagatgttttcagtga, ccacagaaaatggggtggga,
atgtggtcatgtgatggttt, agctgcatacatcgctgaaa, catctcaccatagcttttat,
atatttcccaagacccttttt, gatgcagaagaggacagctg, aaacatctgggctggtacag,
cttgatctgtcaatttctcc, tccttagtaatagcaggacc, ggggaatagttcatcgactt,
tttcccagatgaaaagaccc, ctcttctttattgcttcttt, gtcagttcacaatgctatct,
acttcttctccctctctatg, ggccattaaatagctctcta, atgactcaatggagtttcga,
caccatcatcatagcaagga, tgagaaatggatagcaccct, ttcttggtaaccttctttgt,
cctacagaaggctacaggaa, cttcaagaacagaccgtgga, ccatatcttggagtgtgtta,
aacatccatgcaacacttcg, agggaaagccaacaatggct, caagaagggaagggcccaag,
cctaatattagggggacagt, atcaacttggtatccgtttt, atgaaagccaagacagaggt,
ctttcttcacatgcatacgt.

**ELISA to detect soluble CD83.** Supernatants from DC cultures, stimulated with LPS or NSR were harvested and centrifuged at 3,000 xg for 10 min to remove cell debris. Concentrations of soluble CD83 were determined with a commercial ELISA kit (Sino Biological Inc) according to the manufacturers' instructions. Each sample was tested at two different concentrations in triplicate. A standard curve was generated using serial dilutions of recombinant CD83, provided with the kit.

**Proteasome inhibition assay.** DCs were stimulated with different stimulae for 8 h after which clasto Lactacystin β-lactone (CLBL) was added at a final concentration of 5 or 10 µM. The solvent of CLBL, dimethylsulfoxide (DMSO), was used as a control. Sixteen h after these treatments, cells were harvested and analysed by flow cytometry. **Endocytosis inhibition assay.** DCs were stimulated with LPS+NSRmock, LPS+NSR or medium only, for 6 h or 12 h. Cytochalasin D (Sigma) was subsequently added to a final concentration of 10 µg/ml. The solvent of cytochalasin D, dimethylsulfoxide (DMSO), was used as a control. Cells were harvested 6, 12 or 18 h after treatment and analysed by flow cytometry.

RNA isolation and real-time PCR. After stimulation of DCs for 24 h, culture medium was discarded and cells were immediately lysed with Trizol. Total RNA was isolated with Direct-zol™ RNA MiniPrep kit (Zymo research) according to the instructions of the manufacturer. Subsequently, 100 ng RNA of each sample was reverse-transcribed using random primers and Superscript III reverse transcriptase (Promega). Quantitative real-time PCR was performed as previously described (Wichgers Schreur et al., 2011. PloS one 6: e22299). The primer sequences are enlisted in Table 1.

**Statistical analysis.** Differences in CD83 surface expression levels were analysed with Student's T test for paired samples. Differences in mRNA levels of CD80, glyceraldehyde-3-phosphate dehydrogenase (GAPDH) and peptidylprolyl isomerase A (PPIA) and differences in CD83 expression in the endocytosis inhibition assay were analysed using Student's T test. Values of p<0.05 were considered significant. Analyses were performed with GraphPad Prism^{®} 5.04 software.

**Table 1**

| Sequences of the primers used for quantification of CD83, CD80, CD40, CD86 and GAPDH mRNAs by real-time PCR | | |
|---|---|---|
| **mRNA** | **Forward primer** | **Reverse primer** |
| CD83 | ATGGAGACACCCCAGGAAGAC | TCAGGGAATAGGGCCTTTCA |
| CD80 | CCTCAATTTCTTTCAGCTCTTGGT | GGACAGCGTTGCCACTTCTT |
| CD40 | GGCAAAGAGAGTCGCATCTCA | CCAGGCTCTCTGGCCAACT |
| CD86 | AGTTGGACCCTGAGACTTCACAA | GGTGGATGCGAATCATTCCT |
| GAPDH | CCATCTTCCAGGAGCGAGATC | GCCTTCTCCATGGTGGTGAA |

### Results

### Phenotypic characterisation of DCs after infection with NSR.

Immature dendritic cells (iDCs) were generated as described in materials and methods and were inoculated with NSR particles expressing GFP (Fig. 1A). Initial experiments revealed that GFP expression in DC cultures was detectable already at 6 h post infection (hpi, data not shown). Infection efficiencies varied between different experiments and donors, but under optimal conditions exceeded 90% (Fig. 1B).

Monitoring of NSR infection over time revealed that the number of GFP-positive (GFP+) DCs increased gradually until 24 hpi (Fig. 1C). After that time point, a rapid decrease was noticed and at 48 hpi a 5-fold reduction was observed, compared to GFP+ cell counts at 24 hpi. The decrease in GFP+ cells coincided with the decrease in the total numbers of viable cells. Infection of DCs with NSR resulted in distinct morphological changes of the cells. Infected DCs acquired flat and stretched shapes, discriminating them from untreated cells, which remained predominantly round-shaped (Fig. 1D). The phenotype of the infected cells resembled closely that of cells treated with LPS or a combination of LPS and poly(I:C), which are known to trigger DCs maturation (Banchereau and Steinman, 1998. Nature 392: 245-252). This finding suggests that NSR infection of DCs results in maturation. **Cytokines secreted by infected DCs.** To analyse the secretion of proinflammatory cytokines, NSR infection was performed such that more than 90% of the cells were positive for GFP. Analysis of supernatants, harvested at 24 hpi revealed significant secretion of IFN-γ, TNF-α and IL-6 from NSR-infected DCs as compared to control NSRmock-infected cells. In contrast, concentrations of IL-10, which is known for its immunosuppressive functions remained low (Fig. 2).

### Surface expression of maturation markers on NSR-infected DCs.

Maturation of DCs is associated with upregulation of the surface expression of MHC class I and II molecules and co-stimulatory molecules such as CD80, CD86 and CD40 (Banchereau et al., 2000. Ann Rev Immunol 18: 767-811; Mellman and Steinman, 2001. Cell 106: 255-258). The hallmark of mature human DCs is the de novo presentation of CD83 at the cell surface. To test the maturation status of infected DCs, we analysed surface expression of MHC-I, MHC-II, CD40, CD80, CD83 and CD86 molecules upon infection. To monitor any unspecific effect that the NSR inoculum might have on DC maturation, two controls were included. The first control, denoted NSRmock, consisted of a similarly prepared inoculum lacking NSR particles. The second control, denoted NSRinact, contained UV-inactivated NSR particles. As a positive control for DC maturation, LPS was used. Untreated DCs were used as a reference.

Flow cytometry analysis of infected, GFP+ DCs at 24 hpi revealed a strong induction of CD80 surface expression when compared to untreated cells or cells incubated with NSRmock or NSRinact (Fig. 3, left and middle panels). A less prominent but still distinct increase was observed in CD40 and MHC-II surface expression, while upregulation of CD86 and MHC-I was minor. Expression levels of all markers in NSR-infected cells resembled (CD80, CD86, MHC-I) or slightly exceeded (CD40, MHC-II) those observed in LPS-stimulated cells. Strikingly, although CD83 expression was strongly upregulated 24 h after LPS stimulation, no upregulation of CD83 was observed in GFP+ cells at this time point.

To address further the dramatic difference of CD83 levels between LPS-treated and NSR-infected cells, LPS stimulation was performed simultaneously with NSR infection or with control NSRmock or NSRinact incubation. Combination of LPS with NSRmock or NSRinact resulted in upregulation of CD83 at the cell surface comparable to that observed after treatment of DCs with LPS only (Fig. 3, right panels). However, incubation of DCs with both LPS and NSR did not result in upregulation of CD83. Expression levels of CD83 remained significantly lower than those in cells co-incubated with LPS and NSRmock control. In contrast, co-inoculation with LPS and NSR resulted in additional upregulation of CD40, CD80, MHC-I and MHC-II surface expression. The levels of CD86 remained stable and were comparable to those observed after incubation with LPS or NSR. Analysis of DCs that were GFP-negative (GFP-) at 24 hpi, revealed that the levels of all surface molecules were elevated when compared with the negative controls, presumably representing a bystander effect resulting from cytokines released by GFP+ cells (Fig. 3).

The effect of the controls NSRmock and NSRinact on DCs was consistently comparable, therefore in following experiments only NSRmock was used as negative control.

**Kinetics of CD83 expression in NSR-infected DCs.** To investigate the kinetics of CD83 surface expression, DCs were harvested after 4, 8, 12, 16, 24 and 48 hpi and analysed with flow cytometry. As a reference marker for upregulation, CD80 was used. NSRmock treated cells did not display changes in CD83 surface expression during the whole observation period, while in LPS treated cells upregulation of CD83 was observed already after 4 h and expression levels remained high until the end of the observation period (Fig. 4, upper panel). Similar expression levels were observed after combined LPS-NSRmock treatment. In NSR infected cells, the GFP signal was detectable at 8 hpi. In GFP+ cells, an initial upregulation of CD83 was observed which peaked at 12 hpi. After that time point, CD83 levels decreased gradually and at 24 hpi reached the levels of the negative control. Contrastingly, in GFP- cells, CD83 expression levels were initially comparable to those of control cells and at 12 hpi levels increased and remained high, revealing that CD83 upregulation in bystander DCs depended on the presence of GFP+ cells. When a combination of LPS and NSR was used to stimulate DCs, maximal upregulation of CD83 in GFP+ cells was reached already at 8 hpi, consistent with the presence of LPS. However, as time progressed, CD83 expression decreased, reaching the lowest levels at 24 hpi. In GFP- cells, CD83 levels resembled those in cells treated with LPS or LPS combined with NSRmock. These data indicate that infection with NSR results in initial upregulation of CD83 that is accelerated by the presence of LPS. Later, as viral genome replication advances, CD83 is gradually depleted from the cell surface, counteracting the effect of LPS. Notably, CD83 was dramatically upregulated at 48 hpi in all stimulated cells, including those positive for GFP.

In contrast to CD83, CD80 displayed a gradual upregulation in cells inoculated with NSR and in cells inoculated with a combination of LPS and NSR, regardless of the expression of GFP. The dynamics of CD80 expression in GFP-positive cells resembled closely that of cells treated with LPS or LPS+NSRmock (Fig. 4, lower panel). A strong increase in the surface expression of CD80 was observed at 48 hpi similar to CD83.

**CD83 mRNA levels and subcellular distribution are not affected by NSR-infection.** To evaluate whether the reduced CD83 surface expression observed after NSR infection correlated with reduced mRNA levels, we analysed with qPCR the quantities of CD83 mRNA in DC lysates prepared 24 h post NSR or LPS+NSR treatments, resulting in more than 90% GFP-positive cells. Levels of two house-keeping gene mRNAs, glyceraldehyde-3-phosphate dehydrogenase (GAPDH) and peptidylprolyl isomerase A (PPIA), and the level of CD80 mRNA were determined as well and served as controls. The results show that neither NSR infection, nor a combination of LPS stimulation and NSR infection significantly affected the total levels of CD83 mRNA, as compared to control treatments (Fig. 5). In contrast, levels of CD80 mRNA were upregulated by both NSR and LPS+NSR treatments, as well as by LPS treatment, respective to the relevant controls. Strikingly, NSR and LPS+NSR incubation resulted in significant decreases in mRNA levels of GAPDH and PPIA.

To be able to discriminate between GFP+ and GFP- in the DC population and to exclude possible arrest of host mRNA nuclear transport, which is a common mechanism used by viruses to counteract cellular antiviral mechanisms (Kuss et al., 2013. Viruses 5:1824-1849), we also evaluated subcellular location of GAPDH, CD80 and CD83 mRNAs using a fluorescence in situ hybridisation (FISH) technique. With this technique, individual mRNA molecules are visualized, revealing their total amount and cellular location. The overall FISH results corresponded very well with the qRT-PCR data (Fig. 6). The significant decrease in the quantity of GAPDH mRNA, detected with qPCR in NSR infected cells, correlated with strongly reduced number of spots, detected with FISH in GFP+ cells. Interestingly, no reduction in GAPDH mRNA was observed in GFP- cells. CD80 mRNAs levels were induced by LPS stimulation, as well as by NSR infection, both in GFP+ and GFP- cells. Analysis of CD83 mRNA revealed similar total number of spots in all treatment conditions and no differences in mRNA distribution. Collectively, the FISH and qPCR data reveal that the downregulation of the CD83 protein observed after NSR infection, did not correlate with downregulation of the corresponding mRNA.

**Surface downregulation of CD83 does not result from release into the growth medium.** Sénéchal and co-workers reported downregulation of CD83 in monocyte-derived mature DCs infected with human cytomegalovirus (Senechal et al., 2004. Blood 103: 4207-4215). The downregulation was attributed to release of the protein into the culture medium. This finding was confirmed by Kummer et al (Kummer et al., 2007. J Virol 81: 6326-6338). Regarding those reports, we investigated whether the decrease of CD83 downregulation in NSR infected cells resulted from shedding of the protein from the cell surface. To this end, supernatants of NSR-infected cells and of cells treated with LPS were harvested at 24 hpi and analysed for the presence of soluble CD83 by ELISA. Quantities of CD83 in the supernatant of LPS stimulated cells were comparable to those previously reported (Fig. 7A) (Kummer et al., 2007. J Virol 81: 6326-6338). No difference in soluble CD83 was found between LPS-treated cells and NSR-infected cells, demonstrating that shedding of CD83 into the growth medium does not explain the observed surface downregulation in NSR-infected DCs.

**Inhibition of the proteasomal or endocytic degradation pathways does not prevent NSR-mediated downregulation of CD83.** Mature human DCs, infected with herpes simplex virus type 1 (HSV-1), were reported to downregulate CD83 by proteasomal degradation (Kummer et al., 2007. J Virol 81: 6326-6338). This process is mediated by the immediateearly gene ICP0 of HSV-1 and is prevented by inhibition of the cellular proteasome machinery. To investigate whether proteasomal degradation of CD83 explains the decreased surface exposure of this molecule after NSR infection, we used the inhibitor clasto Lactacystin β-lactose (CLBL) to supress cellular proteasomal activity. This drug acts selectively and irreversibly on the 20S and 26S subunits of the proteasome without affecting serine and cysteine proteases. We preferred CLBL because earlier experiments demonstrated that this drug did not exert negative effect on RVFV replication, as opposed to another often used alternative drug, MG-132 (Habjan et al., 2009. J Virol 83: 4365-4375). As we already showed that CD83 levels increase in the first 8-12h after infection and then gradually decrease, we first incubated DCs with LPS+NSR or LPS+NSRmock for 8 h and then added CLBL in two different concentrations. DMSO, the solvent of CLBL, was used as a control. Analyses were performed at 24 hpi. Addition of CLBL or DMSO to LPS+NSR stimulated cells did not have an effect on CD83 surface expression as compared to LPS+NSRmock stimulated cells (Fig. 7B).

Stable levels of CD83 at the DC surface are maintained by continuous production and recycling of the exposed protein in both immature DCs and mature DCs (Klein et al., 2005. Intern Immunol 17:477-487). Inhibition of endocytosis with cytochalasin D was shown to promote surface exposure of CD83 in both DCs types. Cytochalasin D (Cyt D) is a drug that depolymerizes F-actin filaments and thereby blocks endocytosis, without affecting the exocytotic pathway (Mc Dermott et al., 2002. Mol Biol Cell 13: 317-335; Lamaze et al., 1997. J Biol Chem 272: 20332-20335; Durrbach et al., 1996. J Cell Science 109: 457-465). Cyt D treatment of infected cells was therefore employed to investigate possible involvement of endocytic degradation pathways in NSR-mediated downregulation of CD83. LPS+NSR co-stimulated cells, control LPS+NSRmock stimulated cells and untreated cells were incubated with Cyt D. Treatment with Cyt D for 6 h, added 6 h after stimulation resulted in an increase in surface-exposed CD83 in all stimulation conditions. However, in infected cells, this increase was the lowest (Fig. 7C, left panel). Longer incubation with Cyt D (18 h) resulted in a further increase of the CD83 quantities, but CD83 levels in cells co-stimulated with LPS+NSR were significantly lower than those in LPS+NSRmock treated cells (Fig. 7C, middle panel). Importantly, when Cyt D was added at 12 h post treatment, it had no effect on CD83 expression levels in infected cells, while in control cells an increase in expression was observed (Fig. 7C, right panel). The combined results suggest that CD83 downregulation in NSR-infected cells does not involve increased degradation via the proteasomal or endocytic degradation pathways.

**CD83 protein levels are strongly reduced in cell lysates of NSR-infected DCs.** Finally we evaluated levels of total CD83 protein in cell lysates. As CD83 expression was first induced and then downregulated in NSR infected cells, we hypothesized that these alterations may result from trapping of the molecule inside the cells. Alternatively, translational suppression can lead to the observed downregulation. To test these hypotheses, we analysed with SDS-PAGE and Western blotting the quantities of CD83 in cell lysates of NSR-infected cells and LPS+NSR co-stimulated cells and compared these with CD83 quantities in untreated cells, cells stimulated with NSRmock, LPS or LPS+NSRmock at 24 hpi. More than 90% of the infected DCs were positive for GFP. In untreated and NSRmock treated cells, a single band of around 42 kDa was visible, corresponding to the known molecular weight of CD83 when present in intracellular protein pools (Klein et al., 2005. Intern Immunol 17: 477-487) (Fig. 7D). Upon induction with either LPS or LPS+NSRmock, CD83 expression was upregulated as evidenced by the appearance of a lower molecular weight band that corresponds to de novo synthesized CD83, as well as higher molecular weight bands that correspond to higher-glycosylated, surface-exposed protein (Klein et al., 2005. Intern Immunol 17: 477-487). In both NSR and LPS+NSR treated cells, the detected CD83 protein levels closely resembled those in the untreated and NSRmock treated cells and only the band that corresponds to the preformed protein was visible, while the bands corresponding to the de novo form and the high-glycosylated form were not detected. Digestion of cell lysates with peptide-N-Glycosidase F to remove the N-linked carbohydrates revealed a discrete band of deglycosylated protein. Amounts were comparable in untreated, NSRmock, NSR and LPS+NSR treated cells and much higher in cells treated with LPS or LPS+NSRmock. The total CD83 protein quantities in cell lysates correlated well with those measured by flow cytometry. This finding suggests that the observed downregulation of CD83 from the cell surface of NSR infected cells at 24 hpi does not result from trapping of the protein inside the cells, but rather from suppression of protein translation by an as yet unknown mechanism.

### Example 2

To study the T-cell activation capacity of infected DCs, an NSR variant was constructed that encodes the immunodominant NLVPMVATV epitope of the human cytomegalovirus (HCMV) pp65 (pp65₄₉₅₋₅₀₃), fused to the C-terminus of GFP (Fig. 8A). NSR particles encoding this fusion protein (NSR-NLV) and particles encoding GFP only (NSR-GFP) were used to infect DCs obtained from a HLA-A2 positive donor. As a positive control, DCs were incubated with synthetic NLVPMVATV peptide (1 µM) and culture medium was used for negative controls. After an overnight incubation, HLA-A*0201-restricted, NLVPMVATV-specific CD8+ T lymphocytes (Flinsenberg et al., 2012. Blood 120: 5163-5172) were added to the DCs and co-cultured for 4.5 h. Lymphocytes were harvested, stained for surface markers CD3, CD8 and CD107a (LAMP-1) and subsequently intracellularly stained for IFN-γ and TNF-α. The results of this experiment demonstrate that NSR can be successfully used as a vector to deliver specific immunogenic epitopes to human DCs, which trigger an effector function in corresponding CD8+ T cells (Fig. 8B).

To investigate the feasibility of using NSR for cancer immunotherapy, we made use of C57BL/6 Kh (B6, H-2b) mice and E.G7-OVA cells, a chicken ovalbumin (OVA) gene-transfected clone of mouse lymphoma EL4 cells, obtained from the American Type Culture Collection (Manassas, VA, USA). An NSR variant was constructed that encodes the CD8-restricted SIINFEKL epitope of OVA (OVA 257-264) fused to GFP (NSR-OVA) (Fig. 9A). To determine whether NSR-OVA vaccination elicits a cellular immune response specific for this epitope, mice were vaccinated twice, with a week interval, with 10E8 TCID50 (50% tissue culture infective dose) of NSR-OVA (Fig. 9B). Control mice were vaccinated with NSR-GFP. One week after the second vaccination, spleen cells were collected and evaluated for their ability to produce interferon-γ after stimulation with a synthetic SIINFEKL peptide (10 µg/ml, Invitrogen) in an ELISpot assay (Oreshkova et al., 2013. PloS one 8: e77461). This experiment demonstrated that NSR-OVA vaccination elicits a SIINFEKL-specific cellular immune response (Fig. 9C).

We finally asked whether prophylactic or therapeutic vaccination with NSR-OVA can reduce outgrowth of E.G7-OVA tumor cells (Fig. 10A). Mice were subcutaneously inoculated with 10E6 E.G7-OVA cells and euthanized by cervical dislocation when tumor size reached 5,000 mm3. Both prophylactic and therapeutic vaccination with NSR-OVA resulted in increased survival times, as compared to control mice that had received NSR-GFP (Fig. 10B). Therapeutic vaccination resulted in complete tumor clearance in 2/10 mice, whereas prophylactic vaccination resulted in clearance of tumors in 6/10 mice. As expected, re-cultured tumor cells collected from NSR-GFP-vaccinated mice upon necropsy were found to express OVA, as determined by a commercial OVA ELISA (Agro-Bio). Surprisingly, tumor cells collected from mice vaccinated with NSR-OVA did not express detectable levels of OVA (Fig. 10C). This suggests that small numbers of the inoculated cells did not express OVA or lost OVA expression in time, and that tumor cells expressing OVA were efficiently cleared by either prophylactic or therapeutic vaccination.

### Conclusion

The present work demonstrates that NSR particles can successfully deliver an immunogenic peptide to human DCs and that these cells are capable of activating CD8+ T cells. In addition, vaccination of mice with NSR expressing a single CD8-restricted epitope resulted in the complete clearance of lymphoma cells expressing the targeted antigen. Considering that self-antigens are much less immunogenic, NSR particles will be designed to express not only CD8+ peptides, but also CD4+ and B-cell epitopes. We hypothesize that NSR-based vectors are able to break CD4+CD25+ regulatory T cell-mediated tolerance due to innate immune responses resulting from efficient RIG-I activation (Fuertes et al., 2013. Trends Immunol 34: 67-73; Yang et al., 2004. Nature Immunol 5: 508-515).

## Claims

1. An avirulent Phlebovirus-based vector, whereby a gene encoding a tumor-associated antigen is functionally inserted into the genome of said Phlebovirus-based vector.

2. The Phlebovirus-based vector according to claim 1, whereby said gene encoding said antigen is inserted in a NSs encoding region on the S-genome segment.

3. The Phlebovirus-based vector according to claim 1 or claim 2, whereby said gene encoding said antigen replaces a NSs encoding region on the S-genome segment.

4. The Phlebovirus-based vector according to any one of claims 1-3, whereby said vector is a Rift Valley fever virus-based vector.

5. A Phlebovirus-based vector according to any one of claims 1-4, for use in a method of treating an individual suffering from a tumor.

6. A method of presenting at least one antigenic peptide at the surface of an antigen presenting cell (APC), the method comprising:
a) infecting said APC with a Phlebovirus-based vector according to any one of claims 1-4;
b) allowing the APC to present said antigenic peptide.

7. The method according to claim 6, whereby said antigen presenting cell is a dendritic cell.

8. The method according to claim 6 or 7, further comprising activating said APCs to mature into fully functional dendritic cells prior to contacting said APC with a Phlebovirus-based vector.

9. A method of ameliorating and/or treating an individual suffering from a tumor, the method comprising administering a Phlebovirus-based vector according to any one of claims 1-4 to said individual to stimulate an immune response in the individual against said tumor-associated antigen, thereby ameliorating and/or treating said individual.

10. The method according to claim 9, wherein the Phlebovirus-based vector is administered together with immune-stimulants.

11. The method according to claim 9, comprising
providing human antigen processing cells (APCs);
infecting said APCs with the Phlebovirus-based vector; and
administering said infected APCs to said individual together with immune-stimulants.

12. The method according to claim 11, wherein the antigen processing cells (APCs) are isolated from the patient prior to infection with the Phlebovirus-based vector.

13. The method according to claim 11 or 12, wherein the antigen processing cells (APCs) are activated to mature into fully functional dendritic cells, prior to infecting said APCs with a Phlebovirus-based vector.
